(19) Europäisches Patentamt
European Patent Office
Office européen des brevets

(11) Publication number: 0 004 018
B1

(12) **EUROPEAN PATENT SPECIFICATION**

(45) Date of publication of patent specification: 09.06.82

(51) Int. Cl.³: C 07 F 7/08, A 61 K 31/695

(21) Application number: 79100571.3

(22) Date of filing: 26.02.79

(54) **1-Oxa-4-aza-2,6-disilacyclohexanes, their production and use as muscle relaxants and pharmaceutical compositions containing them.**

(30) Priority: 06.03.78 US 883886
30.11.78 US 965021

(43) Date of publication of application:
19.09.79 Bulletin 79/19

(45) Publication of the grant of the patent:
09.06.82 Bulletin 82/23

(84) Designated Contracting States:
BE CH DE FR GB LU NL SE

(56) References cited:
US - A - 2 567 131

CHEMISCHE BERICHTE, Jahrgang 96, nr. 4, pages 965—975
* Page 974, 2nd last paragraph; page 975, paragraphs 1,2 *

(73) Proprietor: SANDOZ AG
Lichtstrasse 35
CH-4002 Basel (CH)

(72) Inventor: Barcza, Sandor
8, Larchdell Way
Mountain Lakes 8 NY 07046 (US)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 1-Oxa-4-aza-2,6-disilacyclohexanes, their production and use as muscle relaxants and pharmaceutical compositions containing them

The invention provides compounds of formula I,

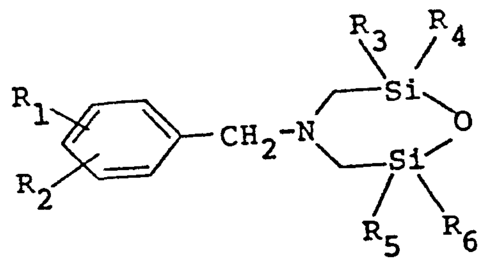

I

in which either

$R_1$ and $R_2$ are independently hydrogen, fluorine, chlorine, alkyl or alkoxy of 1 to 4 carbon atoms, or trifluoromethyl, or

$R_1$ is $-(CH_2)_nN(R_7)(R_8)$, in which $R_7$ and $R_8$ are independently hydrogen, methyl or ethyl, and n is 0 or 1, and

$R_2$ is hydrogen, and

$R_3$, $R_4$, $R_5$ and $R_6$ are independently methyl or ethyl.

The invention also provides a process for the production of compounds of formula I, comprising condensing a compound of formula II,

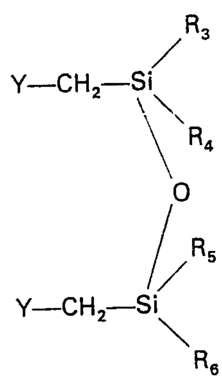

II

in which $R_3$, $R_4$, $R_5$ and $R_6$ are s defined above, and the Y's each represent a leaving group, with a compound of formula III,

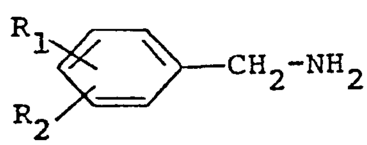

III

in which $R_1$ and $R_2$ are as defined above.

Suitable leaving groups Y include alkyl or aryl-sulphonate groups, e.g. tosylate and mesylate groups, and halogen atoms, e.g. iodo, bromo or chloro. Preferably, the groups Y are identical.

The process may be carried out in conventional manner, suitably in the presence of an acid binding agent, such as a tertiary amine, e.g. pyridine, triethylamine or diisopropylamine, or an alkali metal hydroxide or hydride, such as potassium or sodium hydroxide, or lithium hydride. Alternatively, an excess of the compound of formula III may be employed to serve as acid binding agent. Preferably, no solvent is employed, but, if desired, an inert aprotic solvent such as acetonitrile, dimethylformamide, or dimethylacetamide, or a protic solvent, such as a $C_{1-4}$ alkanol, e.g. methanol or ethanol, may be used. The process is conveniently effected at a temperature of from −10° to +100°C, preferably 20° to 30°C, and the reaction time may vary typically from 2 to 12 hours, more usually about 3 to 5 hours.

The resulting compounds of formula I may be isolated and purified using conventional techniques. Where required, free base forms of the compounds may be converted into acid addition salt forms in conventional manner, and *vice versa*.

The compounds of formula II and III are either known or may be prepared in conventional manner from available materials.

The compounds of formula I possess pharmacological activity. In particular, they possess muscle relaxant activity, as indicated 1) by their activity in the rotorod test as described by Dunham and Miya [J. Am. Pharm. Assoc., *45*, 208, (1957)], 2) by their ability to depress spinal reflexes measured by flexor and patellar responses using force displacement transducers in male cats given 0.1 to 3.0 mg/kg of animal body weight, i.v. of the test compound, and 3) by their ability to produce docility in behaviour tests in mice given 25 to 200 mg/kg of animal body weight, i.p. of the test compound according to the 30-word adjective check sheet system basically as described by Irwin S. (Gordon Research Conference, Medicinal Chemistry, 1959), and Chen (Symposium on Sedative and Hypnotic Drugs, Williams and Wilkins, 1954).

The compounds are therefore indicated for use as muscle relaxants. An indicated suitable daily dosage is from 5 to 500 mg, suitably administered in divided doses of from 1.25 to 250 mg two to four times daily, or in sustained release form.

The compounds may be administered in free base form, or in the form of physiologically acceptable acid addition salts, which salt forms have the same order of activity as the free base forms. Suitable salt forms include mineral acid salt forms, e.g. the hydrochloride, hydrobromide or sulphate.

The compounds may be administered alone, or in admixture with a pharmaceutically acceptable diluent or carrier, and, optionally, other excipients, and administered orally in such forms as tablets, elixirs, capsules or suspensions, or parenterally in such forms as injectable solutions or suspensions.

The preferred compounds of formula I are those in which $R_3$, $R_4$, $R_5$ and $R_6$ each signify methyl. Particularly preferred are those in which one of $R_1$ and $R_2$ is hydrogen and the other is $C_{1-4}$ alkoxy, more particularly methoxy. The most preferred compound is that of Example 2 hereinafter.

The following Examples illustrate the invention.

## Example 1

4-Benzyl-2,2,6,6-tetramethyl-1-oxa-4-aza-2,6-disilacyclohexane

An amount of 33.6 g (0.315 mole) of benzylamine is added over a period of approximately 5 minutes to 41.4 g (0.1 mole) of 1,3-bis-iodomethyl-1,1,3,3-tetramethyldisiloxane, with stirring, in a bath at room temperature. Stirring is continued at room temperature for one hour. (A slurry results). Addition of 50 ml of acetonitrile gives a clear solution which is then heated to reflux for 2 hours. The cooled solution is distributed between approximately 0.5 l of pentane and 1.5 l of water. The aqueous layer is then extracted with one or more approximately 200 ml portions of pentane, and the combined organic layer is washed twice with approximately 300 ml of water, dried over anhydrous sodium sulphate, filtered and evaporated *in vacuo* to provide a clear yellow mobile liquid. This product is then vacuum distilled to give 4-benzyl-2,2,6,6-tetramethyl-1-oxa-4-aza-2,6-disilacyclohexane, b.p. 134° to 135°C, at 13 mm Hg.

The hydrochloride salt of the title compound is prepared by dissolving 10.0 g (37.7 m moles) of the free base in 150 ml of diethylether. There is then injected, with stirring and ice-cooling, 900 ml of hydrogen chloride gas. The resulting slurry is allowed to stand at 0° overnight, filtered and the precipitate is washed with more ether and dried at 110°C *in vacuo* to give the hydrochloride salt form, m.p. 250 to 251°C.

## Example 2

4-(m-Methoxybenzyl)-2,2,6,6-tetramethyl-1-oxa-4-aza-2,6- disilacyclohexane

A solution containing 92.4 g (400 m moles) of 1,3-bis-chloromethyl-1,1,3,3-tetramethyldisiloxane and 100 g of acetonitrile, dried over molecular sieves, is heated to 90°C bath temperature. To this solution, there is added, with stirring, a solution of 55 g (400 m moles) of *m*-methoxybenzylamine in 88.8 g ($2 \times 1.1 \times 400 = 880$ m moles) of triethylamine, over a period of 25 minutes. A solid separates in the reaction mixture from the time at which about half the amine is added. The mildly exothermic reaction keeps the mixture refluxing. The resulting slurry is stirred for an additional 4-3/4 hours, then cooled in ice. Toluene (~ 0.5 l) is then added and the mixture is extracted with 1 litre of water; the latter is washed with 100 ml of toluene. The combined organic phase is washed with two 500 ml portions of water and concentrated *in vacuo* first at 40°C then at 60°C. The resultant oil is distilled through a short Vigreux column to give 4-(*m*-methoxybenzyl)-2,2,6,6-tetramethyl-1-oxa-4-aza-2,6-disilacyclohexane.

The hydrochloride salt of the title compound is prepared in the following manner.

A solution of 13.5 ml of aqueous concentrated hydrochloric acid (slight excess over 135 m mol) in 50 ml of acetone is added, without cooling, to a solution of 40 g (135 m mol) of 4-(*m*-methoxybenzyl)-2,2,6,6-tetramethyl-1-oxa-4-aza-2,6-disilacyclohexane in 100 ml of acetone. An additional 50 ml of acetone is added to facilitate stirring. The resultant white slurry is stirred for 10 minutes, then 100 ml of ethyl acetate is added, while continuing stirring for an additional 10 minutes. The mixture is then allowed to stand at 0°C for 3 hours. The resulting slurry is then filtered and the precipitate is washed with 60 ml of 1:1 acetone:ethyl acetate, then with 50 ml of ethyl acetate; the solids are resuspended in

each portion of wash. The resulting product is dried overnight *in vacuo* at 100°C to give the hydrochloride salt form, m.p. 230° to 231.5°C.

Examples 3 to 13

In manner analogous to that of Example 1, employing appropriate starting materials in approximately equivalent amounts, the compounds of formula I, set out in the following Table, may be obtained.

| No. | $R_1$ | $R_2$ | $R_3$ | $R_4$ | $R_5$ | $R_6$ | Salt Form | m.p. °C |
|---|---|---|---|---|---|---|---|---|
| 3 | H | *p*-Cl | $CH_3$ | $CH_3$ | $CH_3$ | $CH_3$ | | |
| 4 | H | *p*-F | $CH_3$ | $CH_3$ | $CH_3$ | $CH_3$ | HCl | 247—248° |
| 5 | H | *P*—$CH_3$ | $CH_3$ | $CH_3$ | $CH_3$ | $CH_3$ | HCl | 245—246° |
| 6 | H | *p*-$CH_3O$ | $CH_3$ | $CH_3$ | $CH_3$ | $CH_3$ | HCl | 223° |
| 7 | 3-Cl | 4-Cl | $CH_3$ | $CH_3$ | $CH_3$ | $CH_3$ | | |
| 8 | H | 3-$CF_3$ | $CH_3$ | $CH_3$ | $CH_3$ | $CH_3$ | HCl | 233—234° |
| 9 | H | 4-$(CH_3)_2N$— | $CH_3$ | $CH_3$ | $CH_3$ | $CH_3$ | | |
| 10 | H | 4-$(CH_3)_2NCH_2$— | $CH_3$ | $CH_3$ | $CH_3$ | $CH_3$ | | |
| 11 | H | *m*-Cl | $CH_3$ | $CH_3$ | $CH_3$ | $CH_3$ | HCl | 273° |
| 12 | H | *m*-$CH_3$ | $CH_3$ | $CH_3$ | $CH_3$ | $CH_3$ | HCl | 250° |
| 13 | H | *o*-Cl | $CH_3$ | $CH_3$ | $CH_3$ | $CH_3$ | HCl | 221—222° |

**Claims**

1. Compounds of formula I,

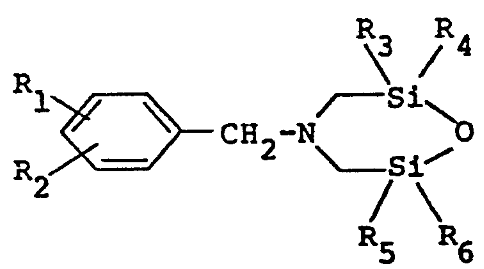

I

in which either

$R_1$ and $R_2$ are independently hydrogen, fluorine, chlorine, alkyl or alkoxy of 1 to 4 carbon atoms, or trifluoromethyl, or

$R_1$ is —$(CH_2)_nN(R_7)$ $(R_8)$, in which $R_7$ and $R_8$ are independently hydrogen, methyl or ethyl, and n is 0 or 1, and

$R_2$ is hydrogen, and

$R_3$, $R_4$, $R_5$ and $R_6$ are independently methyl or ethyl,

in free base or acid addition salt form.

2. Compounds of Claim 1, in which $R_3$, $R_4$, $R_5$ and $R_6$ are all methyl.

3. Compounds of Claims 1 or 2, in which one of $R_1$ and $R_2$ is hydrogen.

4. 4-Benzyl-2,2,6,6-tetramethyl-1-oxa-4-aza-disilacyclohexane, or an acid addition salt thereof.

5. 4-(*m*-Methoxybenzyl)-2,2,6,6-tetramethyl-1-oxa-4-aza-2,6-disilacyclohexane, or an acid addition salt thereof.

6. A compound of Claim 1, in which $R_3$, $R_4$, $R_5$ and $R_6$ are all methyl, $R_1$ is hydrogen and $R_2$ is *p*-fluoro-, *p*-methyl or *p*-methoxy, *m*-chloro, *m*-methyl or *o*-chloro, or an acid addition salt thereof.

7. A process for the production of a compound of formula I, stated in Claim 1, or an acid addition salt thereof, comprising condensing a compound of formula II,

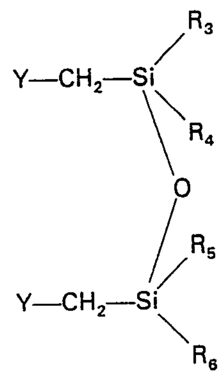

II

in which $R_3$, $R_4$, $R_5$ and $R_6$ are as defined above, and the Y's each represent a leaving group, with a compound of formula III,

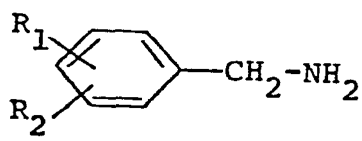

III

in which $R_1$ and $R_2$ are as defined above.

8. A pharmaceutical composition comprising a compound of formula I, stated in Claim 1, in free base or physiologically acceptable acid addition salt form, in association with a pharmaceutically acceptable diluent or carrier.

9. A compound of formula I as claimed in claim 1 for use as a muscle relaxant.

**Revendications**

1. Composés de formule I,

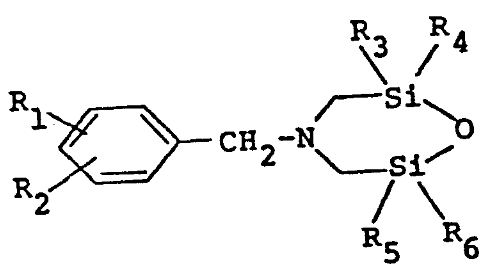

(I)

dans laquelle soit

$R_1$ et $R_2$ représentent, indépendamment l'un de l'autre, l'hydrogène, le fluor, le chlore, un radical alkyle ou alcoxy contenant de 1 à 4 atomes de carbone, ou trifluorométhyle, soit

$R_1$ est un radical $—(CH_2)_nN(R_7)(R_8)$, où $R_7$ et $R_8$ signifient, indépendamment l'un de l'autre, l'hydrogène, un radical méthyle ou éthyle et n signifie 0 ou 1, et

$R_2$ est l'hydrogène, et

$R_3$, $R_4$, $R_5$ et $R_6$ signifient, indépendamment l'un de l'autre, un radical méthyle ou éthyle, sous forme de base libre ou de sel d'addition d'acides.

2. Composés selon la revendication 1, caractérisés en ce que $R_3$, $R_4$, $R_5$ et $R_6$ signifient tous un radical méthyle.

3. Composés selon les revendications 1 ou 2, caractérisés en ce que l'un des substituants $R_1$ et $R_2$ est l'hydrogène.

4. Le 4-benzyl-2,2,6,6-tétraméthyl-1-oxa-4-aza-2,6-disilacyclohexane, ou un sel d'addition d'acides de ce composé.

5. Le 4-(m-méthoxybenzyl)-2,2,6,6-tétraméthyl-1-oxa-4-aza-2,6-disilacyclohexane, ou un sel d'addition d'acides de ce composé.

6. Un composé selon la revendication 1, caractérisé en ce que $R_3$, $R_4$, $R_5$ et $R_6$ signifient tous un radical méthyle, $R_1$ est l'hydrogène et $R_2$ est p-fluoro-, p-méthyle ou p-méthoxy, m-chloro, m-méthyle ou o-chloro, ou un sel d'addition d'acides de ce composé.

7. Un procédé de préparation d'un composé de formule I, spécifié à la revendication 1, ou d'un sel d'addition d'acides de ce composé, comprenant la condensation d'un composé de formule II

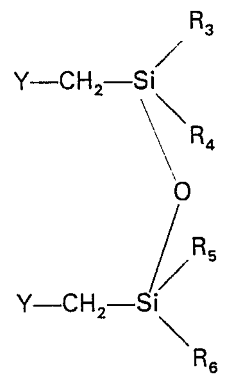

II

dans laquelle $R_3$, $R_4$, $R_5$ et $R_6$ sont tels que définis ci-dessus, et les symboles Y représentent chacun un groupe susceptible d'être éliminé,
avec un composé de formule III

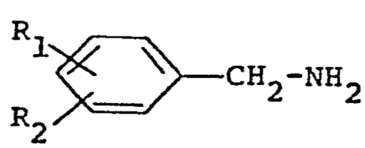

(III)

dans laquelle $R_1$ et $R_2$ sont tels que définis ci-dessus.

8. Une composition pharmaceutique contenant un composé de formule I, spécifié à la revendication 1, sous forme de base libre ou de sel d'addition d'acides acceptable du point de vue physiologique, en association avec un diluant ou un excipient acceptables du point de vue pharmaceutique.

9. Un composé de formule I tel que revendiqué à la revendication 1, pour l'utilisation comme relaxant musculaire.

**Patentansprüche**

1. Verbindungen der Formel I

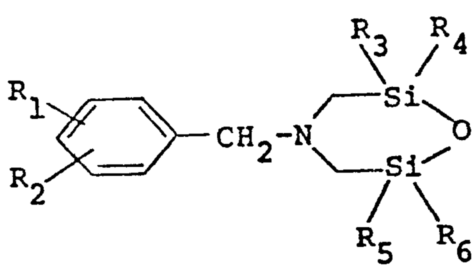

I

in der entweder
$R_1$ und $R_2$ unabhängig Wasserstoff, Fluor, Chlor, Alkyl oder Alkoxy mit 1 bis 4 Kohlenstoffatomen oder Trifluormethyl sind, oder

$R_1—(CH_2)_nN(R_7)(R_8)$ ist, worin $R_7$ und $R_8$ unabhängig Wasserstoff, Methyl oder Aethyl sind, und $n = 0$ oder 1 ist, und

$R_2$ Wasserstoff ist, und

$R_3$, $R_4$, $R_5$ und $R_6$ unabhängig Methyl oder Ethyl sind, in Form der freien Base oder eines Säureadditionssalzes.

2. Verbindungen des Anspruchs 1, in der $R_3$, $R_4$, $R_5$ und $R_6$ alle Methyl sind.

3. Verbindungen des Anspruchs 1 oder 2, in der eines von $R_1$ und $R_2$ Wasserstoff ist.

4. 4-Benzyl-2,2,6,6-tetramethyl-1-oxa-4-aza-2,6-disilacyclohexan oder ein Säureadditionssalz davon.

5. 4-(m-Methoxybenzyl)-2,2,6,6-tetramethyl-1-oxa-4-aza-2,6-disilacyclohexan oder ein Säureadditionssalz davon.

6. Eine Verbindung nach Anspruch 1, worin $R_3$, $R_4$, $R_5$ und $R_6$ alle Methyl sind, $R_1$ Wasserstoff ist und $R_2$ *p*-Fluor, *p*-Methyl oder *p*-Methoxy, *m*-Chlor, *m*-Methyl oder *o*-Chlor sind, oder ein Säureadditionssalz davon.

7. Ein Verfahren zur Herstellung einer Verbindung der Formel I, nach Anspruch 1, oder eines Säureadditionssalzes davon, gekennzeichnet durch die Kondensierung einer Verbindung der Formel II

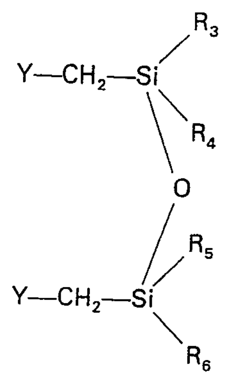

II

in der $R_3$, $R_4$, $R_5$ und $R_6$, wie oben definiert sind, und die beiden Y je eine abspaltbare Gruppe bedeuten, mit einer Verbindung der Formel III

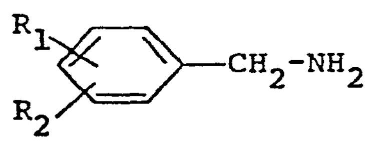

III

in der $R_1$ und $R_2$ wie oben definiert sind.

8. Eine pharmazeutische Zusammensetzung enthaltend eine Verbindung der Formel I, nach Anspruch 1, in Form der freien Base oder eines physiologisch annehmbaren Säureadditionssalzes zusammen mit einem pharmazeutisch annehmbaren Verdünnungsmittel oder Träger.

9. Eine Verbindung der Formel I, wie in Anspruch 1 beansprucht, zur Verwendung als Mukelentspanner.